# EUROPEAN PATENT SPECIFICATION

(11) **EP 1 895 980 B1**
(45) Date of publication and mention of the grant of the patent: **05.12.2012**
(21) Application number: 06772856.8
(22) Date of filing: 12.06.2006
(51) Int. Cl.: A61K 8/34, A61K 8/898, A61K 8/41, A61Q 5/12

(54) **HAIR CONDITIONING COMPOSITION COMPRISING SILICONE POLYMERS CONTAINING QUATERNARY GROUPS**
HAARKONDITIONIERMITTEL MIT SILIKONPOLYMEREN MIT QUATERNÄREN GRUPPEN
COMPOSITION D'APRES-SHAMPOOING COMPRENANT DES POLYMERES DE SILICONE CONTENANT DES GROUPES QUATERNAIRES

(30) Priority: 16.06.2005 US 691175 P; 17.05.2006 US 436167
(43) Date of publication of application: 12.03.2008
(60) Divisional application: 12153301.2
(73) Proprietor: The Procter & Gamble Company, Cincinnati, OH 45202 (US)
(72) Inventor: TORGERSON, Peter, Marte, Washington Court House, Ohio 43160 (US); WILLIAMS, Barbara, Kay, West Chester, Ohio 45069 (US); UEHARA, Nobuaki, Kobe, Hyogo 658-0032 (JP)
(74) Representative: Marollé, Patrick Pierre Pascal
(86) International application number: PCT/US2006/022712
(87) International publication number: WO 2006/138201

(56) References cited:
- WO-A-03/020228
- WO-A2-01/74312
- US-A- 4 833 225
- US-A1- 2004 048 996
- US-A1- 2004 138 400

## Description

### FIELD

The present invention relates to a hair conditioning composition comprising silicone polymers containing quaternary groups and a gel matrix. The composition of the present invention can provide improved conditioning benefits such as smooth feel and reduced friction to both damaged hair and non-damaged hair, while providing other benefits such as slippery and slick feel on wet hair.

### BACKGROUND

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with combing and result in a condition commonly referred to as "flyaway hair," or contribute to an undesirable phenomena of "split ends," particularly for long hair.

A variety of approaches have been developed to condition the hair. A common method of providing conditioning benefit to the hair is through the use of hair conditioning agents such as cationic surfactants and polymers, high melting point fatty compounds, low melting point oils, and silicone compounds. Most of these conditioning agents are known to provide conditioning benefits by depositing on the hair.

Human hair becomes damaged due to, for example, shampooing, combing, permanent waves, and/or coloring the hair. Such damaged hair is often left hydrophilic and/or in a rough condition especially when the hair dries, compared to non-damaged or less damaged hair. There is a need for hair conditioning compositions which provide improved conditioning benefits such as smooth feel and reduced friction on dry hair, especially on damaged hair.

Based on the foregoing, there remains a desire for hair conditioning compositions which provide improved conditioning benefits such as smooth feel and reduced friction on dry hair, especially on damaged hair. There also exists a desire for hair conditioning compositions which provide the above conditioning benefits, while providing other conditioning benefits such as slippery feel and slick feel on wet hair.

### SUMMARY

The present invention provides improved hair conditioning compositions. In accordance with one of the preferred embodiments, there has now been provided a conditioning composition comprising a silicone polymer containing quaternary groups, and a gel matrix. The silicone polymer comprises silicone blocks with greater than about 200 siloxane units. And the gel matrix comprises a cationic surfactant, a high melting point fatty compound, and an aqueous carrier.

Another preferred embodiment includes the composition above combined with at least one additional silicone-based component selected from the group consisting of silicone emulsions, amino silicones, silicone copolyols, and silicone-based quaternary ammonium compounds.

The present invention also provides novel intermediates that are useful for employment in hair compositions. One exemplary intermediate comprises a first polymer comprising silicone and a second polymer comprising a silicone, wherein the resulting combination of the two polymers have a tan δ value of from about 0.01 to about 5.

The present invention further provides unique merchandising systems. The merchandising systems include a hair conditioning composition as provided herein, and a means for communicating a benefit from using the composition. In accordance with one of the preferred embodiments, there has now been provided a merchandising system comprising one of the hair conditioning compositions set forth above; and at least one of packaging for the hair conditioner and marketing material pertaining to the hair conditioner comprising indicia and/or an image providing a communication to consumer related to hair breakage, hair fall out or hair shed, and/or hair strength.

### DETAILED DESCRIPTION

The essential components of the personal care composition are described below. Also included is a nonexclusive description of various optional and preferred components useful in embodiments of the present invention. While the specification concludes with claims that particularly point out and distinctly claim the invention, it is believed the present invention will be better understood from the following description.

All percentages, parts, and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified. The term "weight percent" may be denoted as "wt.%" herein.

All molecular weights as used herein are weight average molecular weights expressed as grams/mole, unless otherwise specified.

The compositions and methods/processes of the present invention can comprise, consist of, and consist essentially of the essential elements and limitations of the invention described herein, as well as any of the additional or optional ingredients, components, steps, or limitations described herein.

Herein, "mixtures" is meant to include a simple combination of materials and any compounds that may result from their combination.

As used herein, "indicia" means an identifying mark, including text and/or graphics.

As used herein, "image" means a photograph, illustration, and/or other pictorial representation of an object.

Preferred embodiments of the hair conditioning composition of the present invention comprise a silicone polymer containing quaternary groups and a gel matrix. These compositions are prepared by a method comprising the step of mixing the silicone polymer containing quaternary groups with the gel matrix.

Damaged hair is less hydrophobic compared to non-damaged and/or less damaged hair. It is believed that by providing improved hydrophobicity to hair, the hair conditioning composition can provide improved smooth feel and reduced friction to the hair. It is also believed that the improved hydrophobicity to the hair can be provided by some other preferred features of the present invention, for example, the use of additional materials such as silicones, and/or cationic surfactants. Further, without being limited to the theory, it is believed that improved hydrophobicity provides improved tolerance to the hair for humidity in the surrounding circumstances, and thus provides reduced frizziness and/or fly-aways on rainy and/or humid days.

The hair conditioning composition of the present invention is preferably substantially free of anionic compounds. Anionic compounds herein include anionic surfactants and anionic polymers. In the present invention, "substantially free of anionic compounds" means that the composition contains 1% or less, preferably 0.5% or less, more preferably less than 0.01% of anionic compounds.

The hair conditioning composition of the present invention has a pH of preferably from about 2 to about 9, more preferably from about 3 to about 7.

### A. Silicone Polymer Containing Quaternary Groups

The compositions of the present invention comprise a silicone polymer containing quaternary groups. The silicone polymer provides improved conditioning benefits such as smooth feel, reduced friction, prevention of hair damage. The silicone polymer is present in an amount of from about 0.1 % to about 15%, preferably from about 0.25% to about 10%, more preferably from about 0.5% to about 5%, and even more preferably from about 2% to about 4% by weight of the composition.

The silicone polymer of the present invention is comprised of at least one silicone block and at least one non-silicone block containing quaternary nitrogen groups, wherein the number of the non-silicone blocks is one greater than the number of the silicone blocks. The silicone polymers correspond to the general structure (I):

A¹-B-(A²-B)ₘ-A¹ (I)

wherein,
B is the silicone blocks with greater than 200 siloxane units;
A² is the non-silicone blocks containing quaternary nitrogen groups;
A¹ are end groups which may contain quaternary groups; and
m is an integer 0 or greater, with the proviso that if m = 0 then the A¹ groups contain quaternary groups.

Structures corresponding to the general formula, for example, are disclosed in U.S. Patent No. 4,833,225, in U.S. Patent Application Publication No. 2004/0138400, and in U.S. Patent Application Publication No. 2004/0048996.

In one embodiment, the silicone polymers can be represented by the following structure (II) 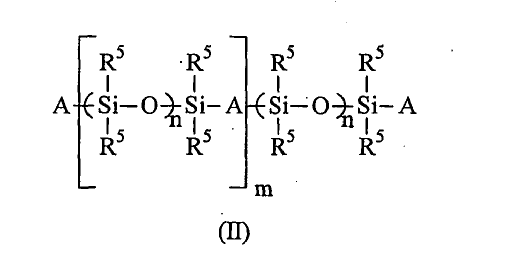 wherein,
A is a group which contains at least one quaternary nitrogen group, and which is linked to the silicon atoms of the silicone block by a silicon-carbon bond, each A independently can be the same or different;
R⁵ is an alkyl group of from about 1 to about 22 carbon atoms or an aryl group; each R⁵ independently can be the same or different;
m is an integer of from 0 or greater, preferably m is less than 20, more preferably m is less than 10; and
n is an integer greater than about 200, preferably greater than about 250, more preferably greater than about 300; preferably less than about 700, more preferably less than about 500.

A preferred structure (III) is with R⁵ as methyl, 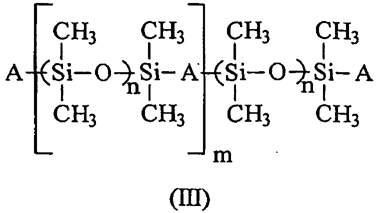 wherein,
A is a group which contains at least one quaternary nitrogen group and is linked to the silicone atoms of the silicone block by a silicon-carbon bond, each A independently can be the same or different;
m is an integer of from 0 or greater, preferably m is less than 20, more preferably m is less than 10;
and n is an integer greater than about 200, preferably greater than about 250, more preferably greater than about 300; preferably less than about 700, more preferably less than about 500.

In another embodiment, the repeat unit of the silicone polymers (the (A²-B) repeat unit in structure (I)) can be represented by the following structure (IV): 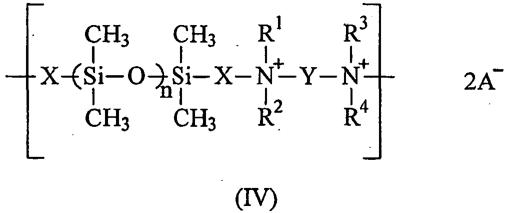 wherein.
X is a bivalent hydrocarbon radical with at least about 4 carbon atoms, which contains a hydroxyl group and can be interrupted by an oxygen atom, and the groups X in the repetition units can be the same or different;
Y is a bivalent hydrocarbon radical with at least about 2 carbon atoms, which can contain a hydroxyl group and which can be interrupted by one or more oxygen or nitrogen atoms, preferably one oxygen atom or one nitrogen atom;
R¹, R2, R³, and R⁴ are the same or different and represent a hydrogen or alkyl groups with from about 1 to about 4 carbon atoms or benzyl groups; in one embodiment, the groups R¹ and R³, or R² and R⁴ are components of a single alkylene group which connects the two N⁺ atoms;
A⁻ is an inorganic or organic anion;
n is an integer greater than about 200, preferably greater than about 250, more preferably greater than about 300; preferably less than about 700, more preferably less than about 500.

In another embodiment, the A¹-B-(A²-B)ₘ-A¹ silicone block copolymer can be described as a polysiloxane compound containing:
a) at least one polyalkylene oxide structural unit with the general structures (V-VIII):

   -A-E-; (V)

   -E-A-: (VI)

   -A-E-A'-: and/or (VII)

   -A'-E-A-. (VIII)

   wherein,
   A is selected from the group consisting of -CH₂C(O)O-, -CH₂CH₂C(O)O-, -CH₂CH₂CH₂C(O)O-,
   -OC(O)CH₂-, -OC(O)CH₂-, -OC(O)CH₂CH₂-, and -OC(O)CH₂CH₂CH₂-;
   A' is selected from the group consisting of -CH₂C(O)-, -CH₂CH₂C(O)-, -CH₂CH₂CH₂C(O)-,
   -C(O)CH₂-, -C(O)CH₂-, -C(O)CH₂CH₂-, and, -C(O)CH₂CH₂CH₂-;
   E is a polyalkylene oxide group selected from the group consisting of -[CH₂CH₂O]_{q}-[CH₂CH(CH₃)O]ᵣ-, and, -[OCH(CH₃)CH₂]ᵣ-[OCH₂CH₂]_{q}-; wherein q is from about 1 to about 200; wherein r is from about 0 to about 200.
   wherein,
   the terminal position oxygen atom of A binds to the terminal position --CH₂-- group of E, and the terminal position carbonyl carbon atom of A' binds to the terminal position oxygen atom of E forming ester groups in each case, and/or at least one terminal position polyalkylene oxide structural unit of the structure (IX)

   -A-E-R² (IX)

   wherein,
   A and E are the same as above; and
   R² is H, straight chain, cyclical or branched C₁ to C₂₀ hydrocarbon group, which can be interrupted by -O--, or -C(O)- and substituted with -OH, and can be acetylene, olefinic, or aromatic;
b) at least one bivalent or trivalent organic group which contains at least one ammonium group;
c) at least one polysiloxane structural unit with the general structure (X)

   --K-S-K--, (X)

   wherein,
   S conforms to the following structure (XI) 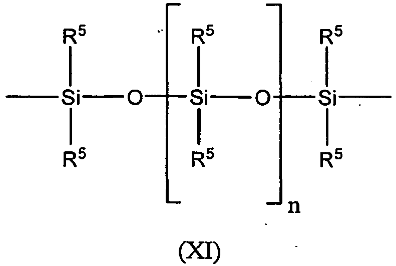 wherein,
   R⁵ is an alkyl group of from about 1 to about 22 carbon atoms or an aryl group, and wherein each R⁵ independently can be the same or different,
   n is an integer greater than about 200, preferably greater than about 250, more preferably greater than about 300; preferably less than about 700, more preferably less than about 500.
   The S groups can be the same or different if several S groups are present in the polysiloxane compound.
   K in structure (X) is a bivalent or trivalent straight chain, cyclical, or branched C₂ to C₄₀ hydrocarbon group which is interrupted by -O-, -NH-, -NR⁵-, -C(O)-, -C(S)-, wherein, 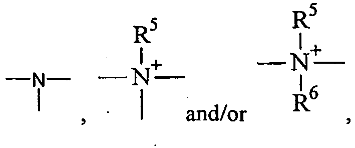 and is substituted by OH;
   R⁵ is as defined above in structure (XI), or represents a bond to a bivalent group R⁶; wherein,
   R⁶ represents a monovalent or bivalent straight chain, cyclical or branched C₁ to C₂₀ hydrocarbon group which is interrupted by -O-, -NH-, -C(O)-, or -C(S)- and can be substituted with -OH or -A-E-R² wherein A, E, and R² are defined as in structure (IX) above.
   The K groups can be identical or different from each other, and in the event K represents a trivalent group, the saturation of the third valence takes place through a bonding to the above mentioned organic group which contains at least one ammonium group;
d) at least one organic or inorganic acid group for neutralization of the charges resulting from the ammonium groups.

A more preferred embodiment is the following structure (XII) 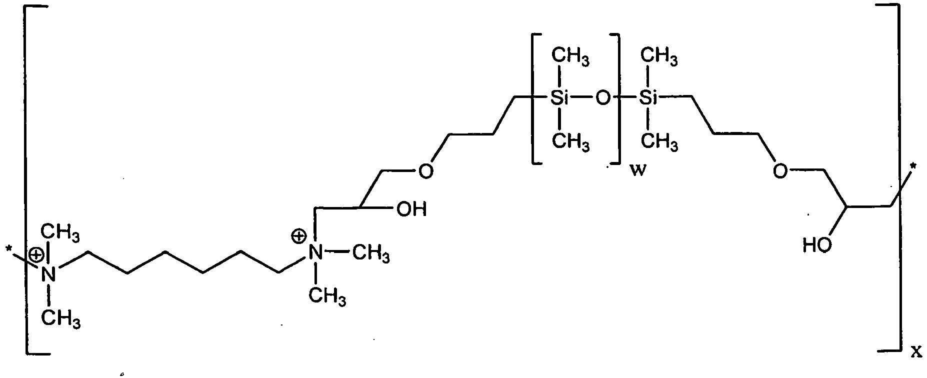 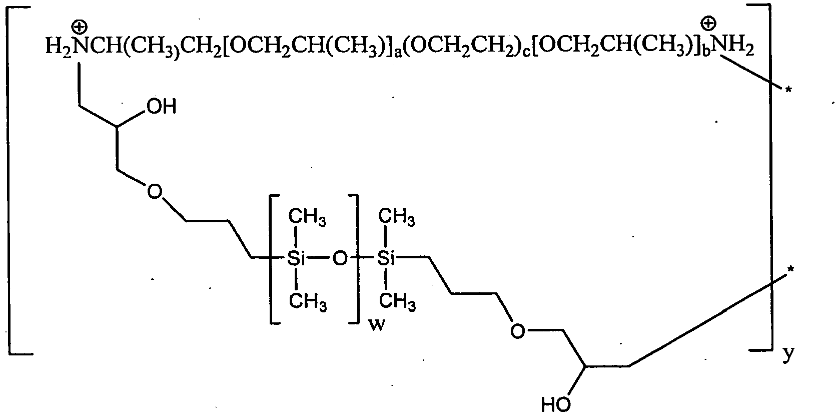 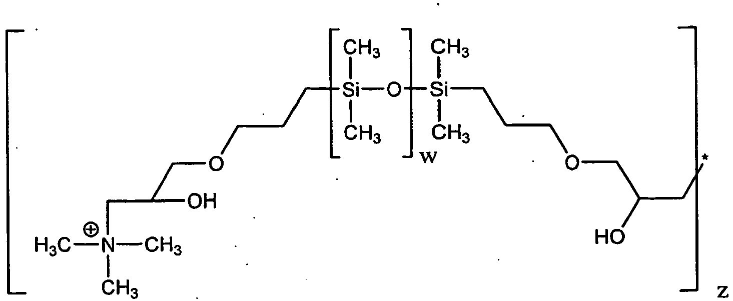 wherein x, y, and z represent mole fractions of the respective components, and hence x + y + Z = 1;
a + b is less than about 200, preferably a + b is less than about 20, more preferably a + b is less than about 10;
c is less than about 200, preferably c is less than about100, more preferably c is less than about 50;
w is an integer greater than about 200, preferably greater than about 250, more preferably greater than about 300; preferably less than about 700, more preferably less than about 500; and
A⁻ is an organic or inorganic anion (for example, the 2A⁻ in the above structure can be laurate and acetate in a 1:1 mole ratio).

### B. Gel Matrix

The composition of the present invention comprises a gel matrix comprising a cationic surfactant, a high melting fatty compound, and an aqueous carrier. The cationic surfactant, together with the high melting fatty compound, and an aqueous carrier, provides a gel matrix which is suitable for providing various conditioning benefits, especially slippery and slick feel on wet hair. Thus, the silicone polymers containing quaternary groups (described above) and the gel matrix both provide conditioning benefits, such that when combined can impart increased functionality as compared to the individual components.

In view of providing the above gel matrix, the cationic surfactant and the high melting point fatty compound are contained at a level such that the mole ratio of the cationic surfactant to the high melting point fatty compound is in the range of, preferably from about 1:1 to about 1:10, more preferably from about 1:2 to about 1:6 or from about 1:1 to about 1:4, in view of providing the above conditioning benefits especially slippery and slick feel on wet hair. Exemplary compositions of the present invention comprise, by weight of the composition, from about 60% to about 99%, preferably from about 70% to about 95%, and more preferably from about 80% to about 95% of a gel matrix including lamellar gel matrix, to which optional ingredients can be added (e.g., silicones).

### 1. Cationic Surfactant

The compositions of the present invention comprise a cationic surfactant. The cationic surfactant is a mono-long alkyl quaternized ammonium salt having the formula (XIII): 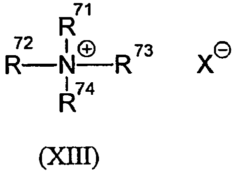 wherein one of R⁷¹, R⁷², R⁷³ and R⁷⁴ is selected from an aliphatic group of from about 16 to about 30 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³, and R⁷⁴ are independently selected from an aliphatic group of from about 1 to about 8 carbon atoms or an aromatic, alkoxy, polyoxyalkylene, alkylamido, hydroxyalkyl, aryl or alkylaryl group having up to about 8 carbon atoms; and X⁻ is a salt-forming anion such as those selected from halogen, (e.g., chloride, bromide), acetate, citrate, lactate, glycolate, phosphate, nitrate, sulfonate, sulfate, alkylsulfate, glutamate, and alkyl sulfonate radicals. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, one of R⁷¹, R⁷², R⁷³, and R⁷⁴ is selected from an alkyl group of from about 16 to about 30 carbon atoms, more preferably from about 18 to about 26 carbon atoms, still more preferably from about 22 carbon atoms; the remainder of R⁷¹, R⁷², R⁷³, and R⁷⁴ are independently selected from the group consisting of CH₃, C₂H₅, C₂H₄OH, CH₂C₆H₅, and mixtures thereof; and (X) is selected from the group consisting of Cl, Br, CH₃OSO₃, and mixtures thereof. It is believed that such mono-long alkyl quaternized ammonium salts can provide improved slippery and slick feel on wet hair, compared to multi-long alkyl quaternized ammonium salts. It is also believed that mono-long alkyl quaternized ammonium salts can provide improved hydrophobicity and smooth feel on dry hair, compared to amine or amine salt cationic surfactants.

Nonlimiting examples of such mono-long alkyl quaternized ammonium salt cationic surfactants include: behenyl trimethyl ammonium chloride available, for example, with tradename Genamine KDMP from Clariant, with tradename INCROQUAT TMC-80 from Croda and ECONOL TM22 from Sanyo Kasei; stearyl trimethyl ammonium chloride available, for example, with tradename CA-2450 from Nikko Chemicals; cetyl trimethyl ammonium chloride available, for example, with tradename CA-2350 from Nikko Chemicals; behenyltrimethylammonium methyl sulfate, available from FeiXiang; hydrogenated tallow alkyl trimethyl ammonium chloride; stearyl dimethyl benzyl ammonium chloride; and stearoyl amidopropyl dimethyl benzyl ammonium chloride.

Among them, more preferred cationic surfactants are those having a longer alkyl group, i.e., C₂₂ alkyl group. Such cationic surfactant includes, for example, behenyl trimethyl ammonium chloride and behenyltrimethylammonium methyl sulfate. It is believed that cationic surfactants having a longer alkyl group provide improved hydrophobicity on dry hair, compared to cationic surfactant having a shorter alkyl group. It is also believed that compared to cationic surfactants having a shorter alkyl group, cationic surfactants having a long alkyl group can provide improved hydrophobicity to the hair, especially to damaged hair, when combined with the polyol esters of the present invention. Alternatively, it is believed that cationic surfactant having an adequate length of alkyl group provides improved slippery and slick feel on wet hair, compared to a cationic surfactant having too long an alkyl group. Thus, it is believed that the selection of C₂₂ alkyl group among long alkyl groups provides balanced benefits between improved hydrophobicity on dry hair and improved slippery and slick feel on wet hair.

The compositions of the present invention preferably comprise the cationic surfactant in amount of from about 0.1 % to about 10%, more preferably from about 1% to about 8%, still more preferably from about 1.5% to about 5% by weight of the composition.

### 2. High Melting Point Fatty Compound

The hair conditioning composition of the present invention comprises a high melting point fatty compound. The high melting point fatty compounds useful herein have a melting point of about 25°C or higher, and are selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than about 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level of from about 0.1 % to about 20%, preferably from about 1% to about 10%, still more preferably from about 2% to about 8%, by weight of the composition.

The fatty alcohols useful herein are those having from about 14 to about 30 carbon atoms, preferably from about 16 to about 22 carbon atoms. These fatty alcohols are saturated and can be straight or branched chain alcohols. Nonlimiting examples of fatty alcohols include cetyl alcohol, stearyl alcohol, behenyl alcohol, and mixtures thereof.

The fatty acids useful herein are those having from about 10 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth-10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e., a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago, Illinois USA), HYSTRENE available from Witco Corp. (Dublin, Ohio USA), and DERMA available from Vevy (Genova, Italy).

### 3. Aqueous Carrier

The hair conditioning composition of the present invention comprises an aqueous carrier. The level and species of the carrier are selected according to the compatibility with other components, and other desired characteristics of the product.

The carrier useful in the present invention includes water and water solutions of lower alkyl alcohols and polyhydric alcohols. The lower alkyl alcohols useful herein are monohydric alcohols having from about 1 to about 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from about 20% to about 95%, preferably from about 30% to about 92%, and more preferably from about 50% to about 90% water.

### C. Additional Components

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, water soluble and water insoluble vitamins such as vitamin A, D, B₁, B₂, B₆, B₁₂, C, biotin, vitamin E with tradename Emix-d available from Eisai, panthenol available from Roche, pantothenic acid, panthenyl ethyl ether available from Roche, and their derivatives; hydrolysed keratin, proteins, plant extracts, and nutrients; emollients such as PPG-3 myristyl ether with tradename Varonic APM available from Goldschmidt, Trimethyl pentanol hydroxyethyl ether, PPG-11 stearyl ether with tradename Varonic APS available from Goldschmidt, Stearyl heptanoate with tradename Tegosoft SH available from Goldschmidt, Lactil (mixture of Sodium lactate, Sodium PCA, Glycine, Fructose, Urea, Niacinamide, Glucosamine, Inositol, Sodium Benzoate, and Lactic acid) available from Goldschmidt, Sodium lactate, Sodium PCA, Glycine, Fructose, Urea, Niacinamide, Glucosamine, Inositol, Sodium Benzoate, Lactic acid, Ethyl hexyl palmitate with tradename Saracos available from Nishin Seiyu and with tradename Tegosoft OP available from Goldschmidt; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes, oxidative dyes and interference pigments; hair oxidizing (bleaching) agents, such as hydrogen peroxide, perborate and persulfate salts, carbonate; hair reducing agents such as the thioglycolates; perfumes; and sequestering agents, such as disodium ethylenediamine tetra-acetate; ultraviolet and infrared screening and absorbing agents such as octyl salicylate; antimicrobial agents; suspending agents; viscosity modifiers; nonvolatile solvents or diluents (water soluble and. insoluble), pearlescent aids, foam boosters, additional surfactants or nonionic cosurfactants, pediculocides, chelants, skin active agents, sunscreens, UV absorbers, and, water soluble and insoluble amino acids such as asparagine, alanin, indole, glutamic acid, tyrosine, tryptamine, and their salts; and antidandruff agents such as zinc pyrithione, pyridinethione salts, azoles, climbazole, octopirox, salicylic acid, selenium sulfide, particulate sulfur, mixtures thereof.

### 1. Silicone

The composition of the present invention may further comprise a silicone compound, in addition to the silicone polymer containing quaternary groups. The silicone compound can be included in an amount of from about 0.1% to about 10%, more preferably from about 0.25% to about 8%, still more preferably from about 0.5% to about 3% by weight of the composition.

The silicone compounds hereof can include volatile soluble or insoluble, or nonvolatile soluble or insoluble silicone conditioning agents. By soluble what is meant is that the silicone compound is miscible with the carrier of the composition so as to form part of the same phase. By insoluble what is meant is that the silicone forms a separate, discontinuous phase from the carrier, such as in the form of an emulsion or a suspension of droplets of the silicone. The silicone compounds herein may be made by conventional polymerization, or emulsion polymerization.

The silicone compounds for use herein will preferably have a viscosity of from about 1,000 to about 2,000,000 centistokes at 25°C, more preferably from about 10,000 to about 1,800,000 centistokes, and even more preferably from about 25,000 to about 1,500,000 centistokes. The viscosity can be measured by means of a glass capillary viscometer as set forth in Dow Corning Corporate Test Method CTM0004, July 20, 1970. Silicone compounds of high molecular weight may be made by emulsion polymerization.

Silicone compounds useful herein include polyalkyl polyaryl siloxanes, polyalkyleneoxide-modified siloxanes, silicone resins, amino-substituted siloxanes, and mixtures thereof. The silicone compound is preferably selected from the group consisting of polyalkyl polyaryl siloxanes, polyalkyleneoxide-modified siloxanes, silicone resins, and mixtures thereof, and more preferably from one or more polyalkyl polyaryl siloxanes.

Polyalkyl polyaryl siloxanes useful here in include those with the following structure (XIV) 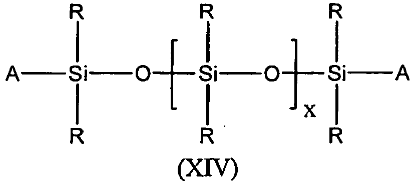 wherein R is alkyl or aryl, and x is an integer from about 7 to about 8,000. A represents groups which block the ends of the silicone chains. The alkyl or aryl groups substituted on the siloxane chain (R) or at the ends of the siloxane chains (A) can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable A groups include hydroxy, methyl, methoxy, ethoxy, propoxy, and aryloxy. The two R groups on the silicon atom may represent the same group or different groups. Preferably, the two R groups represent the same group. Suitable R groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their ViscasilR and SF 96 series, and from Dow Corning in their Dow Corning 200 series. Polymethylphenylsiloxanes, for example, from the General Electric Company as SF 1075 methyl phenyl fluid or from Dow Corning as 556 Cosmetic Grade Fluid, are useful herein.

Also preferred, for enhancing the shine characteristics of hair, are highly arylated silicone compounds, such as highly phenylated polyethyl silicone having refractive index of about 1.46 or higher, especially about 1.52 or higher. When these high refractive index silicone compounds are used, they should be mixed with a spreading agent, such as a surfactant or a silicone resin, as described below to decrease the surface tension and enhance the film forming ability of the material.

Another polyalkyl polyaryl siloxane that can be especially useful is a silicone gum. The term "silicone gum," as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. All of these described references are incorporated herein by reference in their entirety. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof.

Polyalkyleneoxide-modified siloxanes useful herein include, for example, polypropylene oxide modified and polyethylene oxide modified polydimethylsiloxane. These materials are also known as dimethicone copolyols.

Silicone resins, which are highly crosslinked polymeric siloxane systems, are useful herein. The crosslinking is introduced through the incorporation of tri-functional and tetra-functional silanes with mono-functional or di-functional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units, and hence, a sufficient level of crosslinking, such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Preferably, the ratio of oxygen:silicon atoms is at least about 1.2: 1.0. Silanes used in the manufacture of silicone resins include monomethyl-, dimethyl-, trimethyl-, monophenyl-, diphenyl-, methylphenyl-, monovinyl-, and methylvinylchlorosilanes, and tetrachlorosilane, with the methyl substituted silanes being most commonly utilized. Preferred resins are offered by GE Advanced Materials as GE SS4230 and SS4267. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Without being bound by theory, it is believed that the silicone resins can enhance deposition of other silicone compounds on the hair and can enhance the glossiness of hair with high refractive index volumes.

Other useful silicone resins are silicone resin powders such as the material given the CTFA designation polymethylsilsequioxane, which is commercially available as Tospearl^{™} from GE Toshiba Silicones.

Silicone resins can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SiO_{0.5}; D denotes the difunctional unit (CH₃)₂SiO; T denotes the trifunctional unit (CH₃)SiO_{1.5}; and Q denotes the quadri- or tetra-functional unit SiO₂. Primes of the unit symbols, e.g., M', D', T', and Q' denote substituents other than-methyl, and must be specifically defined for each occurrence. Typical alternate substituents include groups such as vinyl, phenyl, amino, hydroxyl, etc. The molar ratios of the various units, either in terms of subscripts to the symbols indicating the total number of each type of unit in the silicone, or an average thereof, or as specifically indicated ratios in combination with molecular weight, complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 10,000.

Amino-substituted siloxanes useful herein include those represented by the following structure (XV) 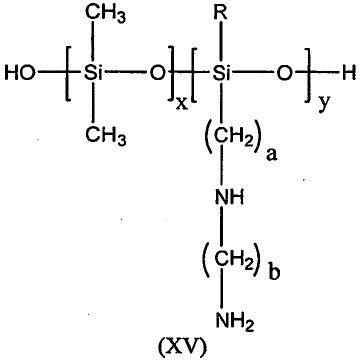 wherein R is CH₃ or OH, x and y are integers which depend on the molecular weight, the average molecular weight preferably being approximately between 5,000 and 10,000; both a and b denote an integer from 2 to 8. This polymer is also known as "amodimethicone".

Suitable amino-substituted siloxane fluids include those represented by the formula (XVI)

(R₁)ₐG₃₋ₐ-Si-(-OSiG2)n-(-OSiG_{b}(R₁)_{2-b})ₘ-O-SiG₃₋ₐ(R₁)ₐ (XVI)

in which G is chosen from the group consisting of hydrogen, phenyl, OH, C₁-C₈ alkyl and preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical of formula CqH_{2q}L in which q is an integer from 2 to 8 and L is chosen from the groups

-N(R₂)CH₂-CH₂-N(R₂)₂;

-N(R₂)₂;

-N(R₂)⁺₃A⁻;

and

-N(R₂)CH₂-CH₂-NR₂H⁺A⁻,

in which R₂ is chosen from the group consisting of hydrogen, phenyl, benzyl, a saturated hydrocarbon radical, preferably an alkyl radical containing from 1 to 20 carbon atoms, and A⁻ denotes a halide ion.

Highly preferred amino silicones are those corresponding to formula (XVI) wherein m=0, a=1, q=3, G=methyl, n is preferably from about 1500 to about 1700, more preferably about 1600; and L is -N(CH₃)₂ or NH₂, more preferably NH₂. Another highly preferred amino silicones are those corresponding to formula (XVI) wherein m=0, a=1, q=3, G=methyl, n is preferably from about 400 to about 600, more preferably about 500; and L is -N(CH₃)₂ or NH₂, more preferably -NH₂. Such highly preferred amino silicones can be called as terminal aminosilicones, as one or both ends of the silicone chain are terminated by nitrogen containing group.

An especially preferred amino-substituted siloxane corresponding to formula (XVI) is the polymer known as "trimethylsilylamodimethicone," of formula (XVII):

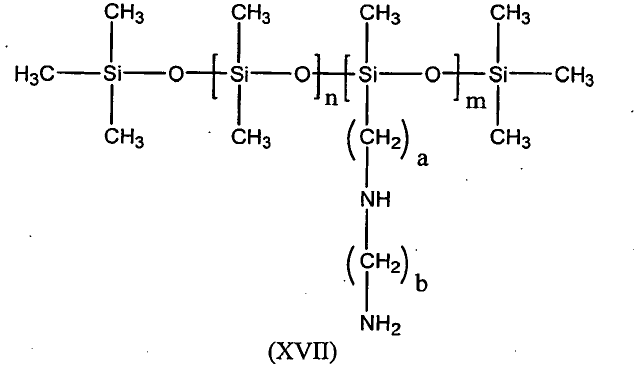

In this formula n and m are selected depending on the molecular weight of the compound desired; both a and b denote an integer from 2 to 8.

In one embodiment of the present invention, the silicone compound is contained in the composition in the form of a silicone emulsion. The silicone emulsion herein is a predispersed stable emulsion comprising at least a surfactant, a silicone compound, and water. The surfactant useful herein is any known to the artisan. Silicone emulsions with a high internal phase viscosity are preferred. One preferred example is HMW2220 with an internal phase viscosity of greater than 120,000,000 centistokes, available from Dow Coming.

Other modified silicones or silicone copolymers are also useful herein. Examples of these include silicone-based quaternary ammonium compounds (Kennan quats) disclosed in U.S. Patent Nos. 6,607,717 and 6,482,969; end-terminal quaternary siloxanes disclosed in German Patent No. DE 10036533; silicone aminopolyalkyleneoxide block copolymers disclosed in U.S. Patent Nos. 5,807,956 and 5,981,681; hydrophilic silicone emulsions disclosed in U.S. Patent No. 6,207,782; and polymers made up of one or more crosslinked rake or comb silicone copolymer segments disclosed in WO2004/062634.

In alternative embodiments of the present invention, the above-noted silicone-based quaternary ammonium compounds may be combined with the silicone polymers described in section A (entitled Silicone Polymer Containing Quaternary Groups) of the instant specification.

### 2. Polysorbate

The hair conditioning composition of the present invention may contain a polysorbate, in view of adjusting rheology. Preferred polysorbate useful herein includes, for example, polysorbate-20, polysorbate-21, polysorbate-40, polysorbate-60, and mixtures thereof. Highly preferred is polysorbate-20.

The polysorbate can be contained in the composition at a level by weight of preferably from about 0.01% to about 5%, more preferably from about 0.05% to about 2%.

### 3. Polypropylene Glycol

Polypropylene glycol useful herein are those having a weight average molecular weight of from about 200 g/mol to about 100,000 g/mol, preferably from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits.

The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair care composition. For example, a rinse-off hair care composition, it is preferred that the polypropylene glycol herein has a solubility in water at about 25°C of less than about 1 g/100 g water, more preferably a solubility in water of less than about 0.5 g/100 g water, and even more preferably a solubility in water of less than about 0.1 g/100 g water.

The polypropylene glycol can be included in the hair conditioning composition of the present invention at a level of, preferably from about 0.01 % to about 10%, more preferably from about 0.05% to about 6%, still more preferably from about 0.1% to about 3% by weight of the composition.

### 4. Low Melting Point Oil

Low melting point oils useful herein are those having a melting point of less than about 25°C. The low melting point oil useful herein is selected from the group consisting of: hydrocarbon having from about 10 to about 40 carbon atoms; unsaturated fatty alcohols having from about 10 to about 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, and glyceryl ester oils; poly α-olefin oils; and mixtures thereof. Preferred low melting point oils herein are selected from the group consisting of: ester oils such as pentaerythritol ester oils, trimethylol ester oils, citrate ester oils, and glyceryl ester oils; poly α-olefin oils; and mixtures thereof,

Particularly useful pentaerythritol ester oils and trimethylol ester. oils herein include pentaerythritol tetraisostearate, pentaerythritol tetraoleate, trimethylolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Exxon Mobil Co.

### 5. Cationic Polymer

Cationic polymers useful herein are those having a weight average molecular weight of at least about 5,000, typically from about 10,000 to about 10 million, preferably from about 100,000 to about 2 million.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol (made by hydrolysis of polyvinyl acetate), maleic anhydride, propylene glycol, and ethylene glycol. Other suitable cationic polymers useful herein include, for example, cationic celluloses, cationic starches, and cationic guar gums.

### 6. Polyethylene Glycol

Polyethylene glycol can also be used as an additional component. The polyethylene glycols useful herein that are especially preferred are PEG-2M wherein n has an average value of about 2,000 (PEG-2M is also known as Polyox WSR® N-10 from Union Carbide and as PEG-2,000); PEG-5M wherein n has an average value of about 5,000 (PEG-5M is also known as Polyox WSR® N-35 and as Polyox WSR® N-80, both from Union Carbide and as PEG-5,000 and Polyethylene Glycol 300,000); PEG-7M wherein n has an average value of about 7,000 (PEG-7M is also known as Polyox WSR® N-750 from Union Carbide); PEG-9M wherein n has an average value of about 9,000 (PEG-9M is also known as Polyox WSR® N-3333 from Union Carbide); and PEG-14M wherein n has an average value of about 14,000 (PEG-14M is also known as Polyox WSR® N-3000 from Union Carbide). As used herein "n" refers to the number of ethylene oxide units in the polymer.

### ELASTICITY AND VISCOSITY

It has been discovered that silicone blends useful in the present invention have a preferred tan δ value. Tan δ is a calculation related to the blended material's elasticity, and is equal to loss modulus (G") divided by storage modulus (G'). Loss modulus (G") and storage modulus (G') are measured by the following method:
Instrument: TA AR2000 or AR-G2 Rheometer, using the oscillation mode with a 40 mm diameter 2 degree AL cone and a gap of 57 µm.
Sample Preparation: scoop samples with spatula, and put the sample onto the measurement table of the rheometer without scrambling to avoid generation of bubbles.
Measurement Protocol: 1) equilibrate sample at a temperature of 26.7°C for 4 minutes; 2) begin oscillation at a frequency step of from 0.1 Hz to 10 Hz with the oscillation fixed at 5.0 Pa and the temperature at 26.7°C. The loss modulus and storage modulus readings are recorded and available via the rheometer instrumentation.

Exemplary silicone blends have tan δ values, as recorded at a frequency of 10 Hz and 26.7°C, from about 0.01 to about 5, preferably from about 0.05 to about 1, and more preferably from about 0.1 to about 0.5. Without being bound by theory, it is believed that the preferred tan δ value correlates to an in-use signal of end rinsing and/or clean feeling after rinsing, and consumers accordingly perceive compositions comprising these silicone blends to be superior in comparison to existing products.

The silicone blends also have preferred viscosity values, including, for example, less than about 10,000,000 centistokes, less than about 1,000,000 centistokes, and less than about 500,000 centistokes. Viscosity can be measured using the rheometer and settings noted above, and using the shear stress controlled mode (0-20 Pa).

Exemplary silicone blends include a combination of silicone polymers selected from those described in sections A and C of the instant specification (including, e.g., combinations that are devoid of the silicone polymers described in section A).

### METHOD OF USE

The hair conditioning compositions of the present invention are used in conventional ways to provide conditioning and other benefits. Such method of use depends upon the type of composition employed but generally involves application of an effective amount of the product to the hair or scalp, which may then be rinsed from the hair or scalp (as in the case of hair rinses) or allowed to remain on the hair or scalp (as in the case of gels, lotions, creams, and sprays). "Effective amount" means an amount sufficient enough to provide a dry conditioning benefit. In general, from about 1g to about 50g is applied to the hair or scalp.

The composition may be applied to wet or damp hair prior to drying of the hair. Typically, the composition is used after shampooing the hair. The composition is distributed throughout the hair or scalp, typically by rubbing or massaging the hair or scalp. After such compositions are applied to the hair, the hair is dried and styled in accordance with the preference of the user. In the alternative, the composition is applied to dry hair, and the hair is then combed or styled in accordance with the preference of the user.

### PRODUCT FORMS

The hair conditioning compositions of the present invention can be in the form of rinse-off products or leave-on products, can be opaque, and can be formulated in a wide variety of product forms, including but not limited to creams, gels, emulsions, mousses and sprays.

### NON-LIMITING EXAMPLES

The compositions illustrated in the following examples exemplify specific embodiments of the compositions of the present invention, but are not intended to be limiting thereof. Other modifications can be undertaken by the skilled artisan without departing from the spirit and scope of this invention.

The compositions illustrated in the following examples are prepared by conventional formulation and mixing methods, an example of which is described below. All exemplified amounts are listed as weight percents and exclude minor materials such as diluents, preservatives, color solutions, imagery ingredients, botanicals, and so forth, unless otherwise specified.

The compositions of the present invention are suitable for rinse-off products and leave-on products, and are particularly useful for making products in the form of a rinse off conditioner.

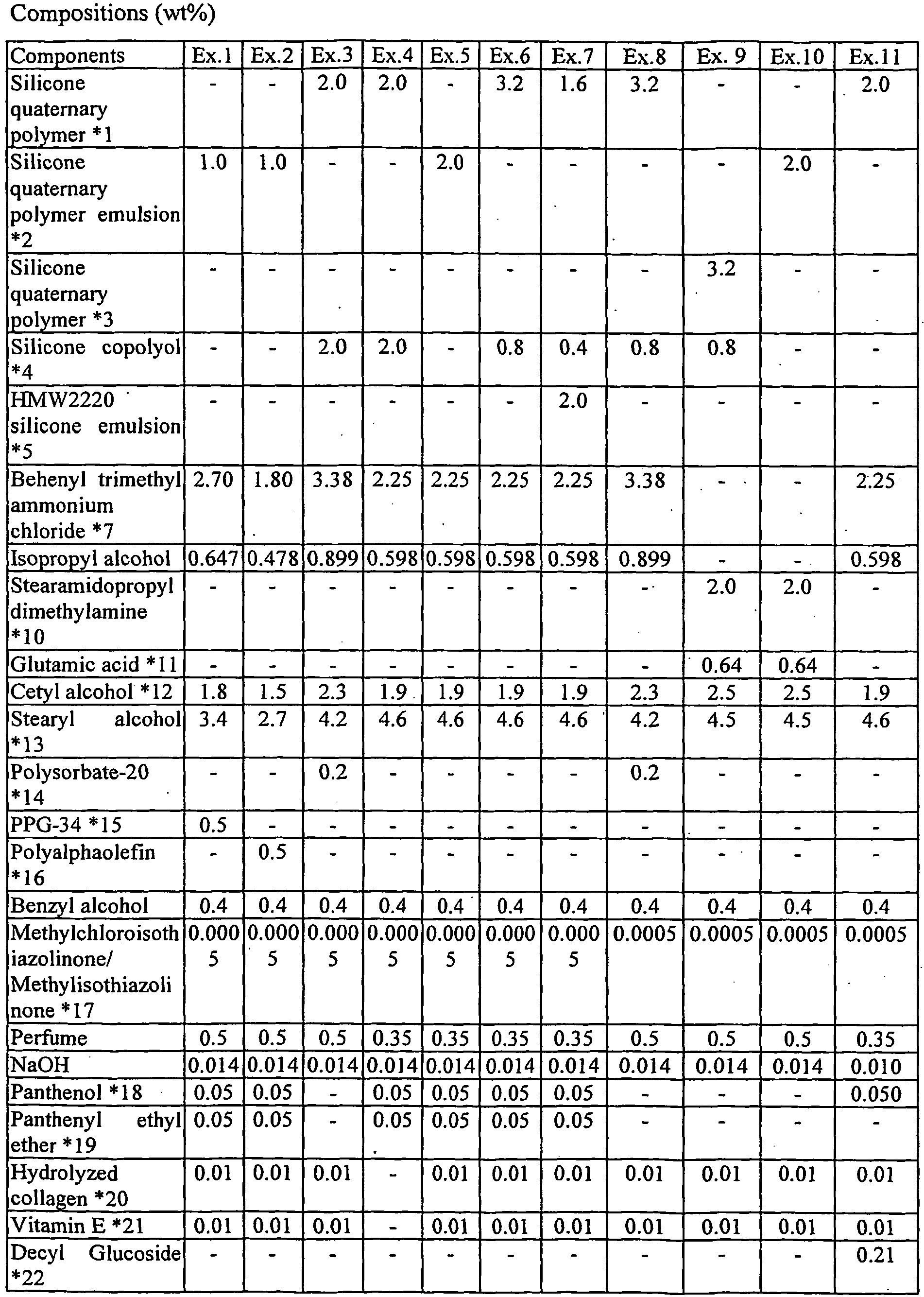

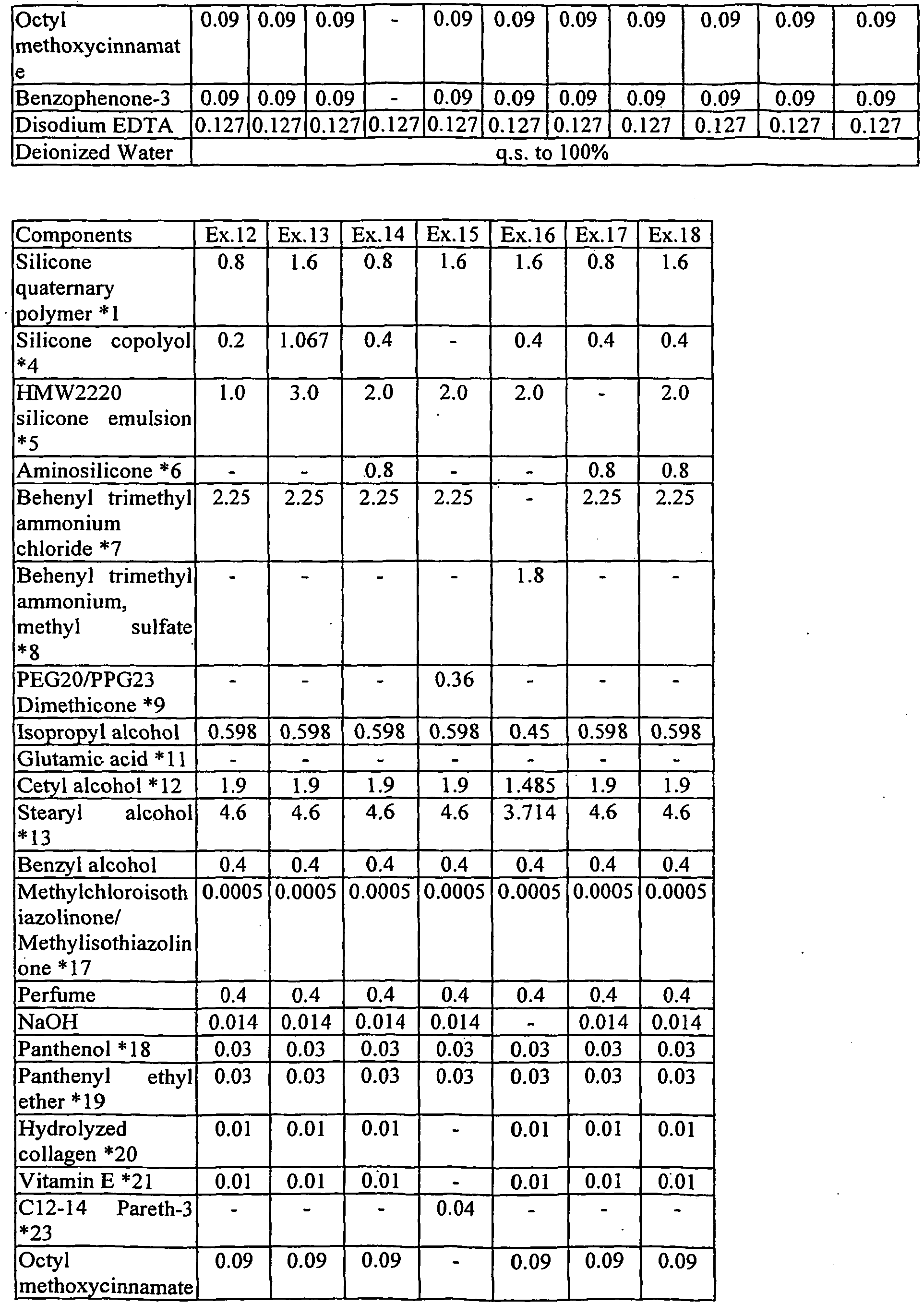

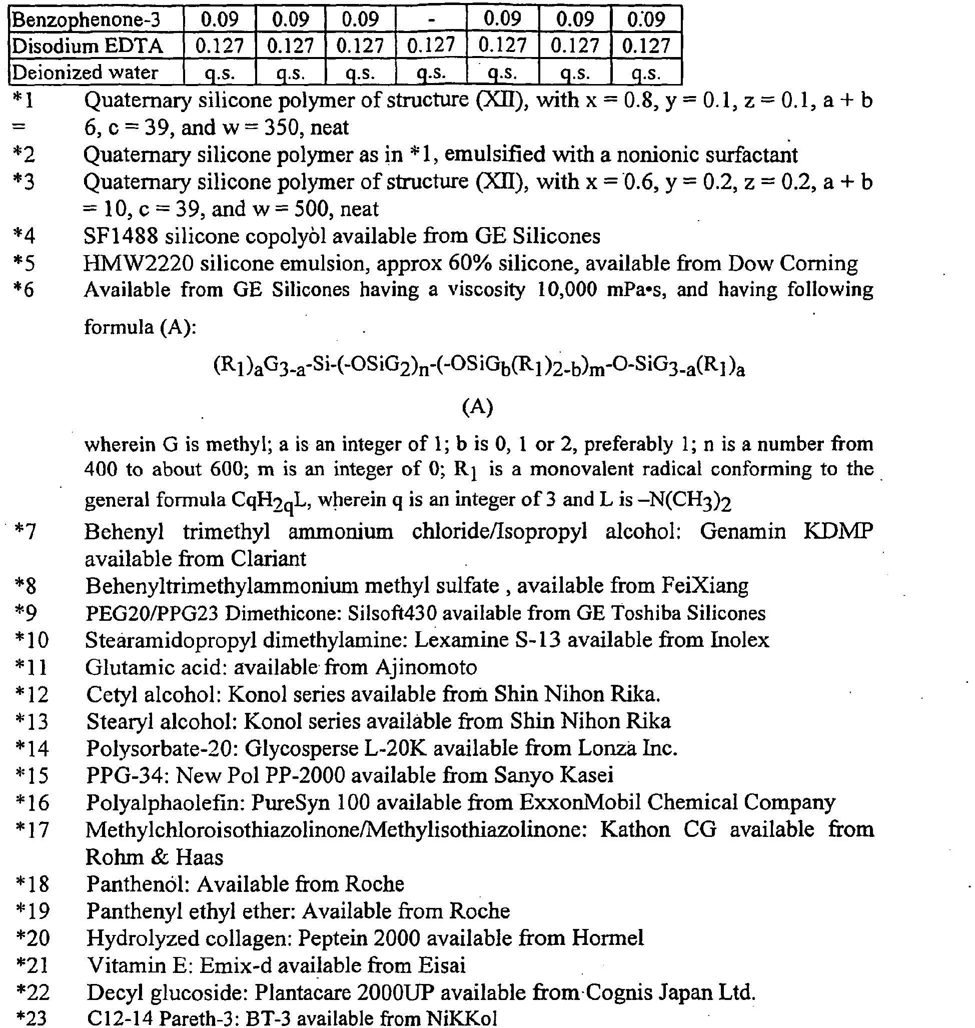

Prepare the hair conditioning compositions by any conventional method well known in the art. They are suitably made as follows:

Make the quaternary silicone polymers as disclosed in U.S. Patent No. 4,833,225, in U.S. Patent Application Publication No. 2004/0138400, and in U.S. Patent Application Publication No. 2004/0048996. The quaternary silicone polymers can be added to the formulation as the neat material, as a preformed emulsion, or as a blend with a lower molecular weight material. In the latter case, the lower molecular weight materials can be any which can form a stable blend with the quaternary silicone polymer. Examples of low molecular weight materials include silicones such as decamethylcyclopentasiloxane, oxygen-containing solvents such as dipropyleneglycol-n-butylether, and silicone copolyols. Silicone copolyols are preferred. For the quaternary silicone polymer emulsion, the polymer is emulsified in the presence of an anionic, cationic, nonionic, or amphoteric surfactant, preferably a nonionic or cationic surfactant, by methods well known in the art. In examples containing the silicone quaternary polymer and a silicone copolyol, the two are mixed prior to addition to the product.

Heat deionized water to 85°C. Mix cationic surfactants and high melting point fatty compounds into the water. Maintain the water at a temperature of about 85°C until the components are homogenized and no solids are observed. Cool the mixture to about 55°C and maintain at this temperature to form a gel matrix. Add the indicated quaternary silicone polymer to the gel matrix, either as the emulstion or as the blend with the copolyol. When included, add polyalphaolefin, polypropylene glycols, silicones, and/or polysorbates to the gel matrix. Maintain the gel matrix at about 50°C during this time with constant stirring to assure homogenization. When included, add other additional components such as perfumes and preservatives at this point also. After homogenization, cool to room temperature.

It should be understood that hair properties and actual/perceived benefits can vary from one individual to another, and therefore, the appended claims are not to be construed as limited to compositions providing all of the benefits that are described herein unless explicitly recited.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A hair conditioning composition, comprising:
a) a silicone polymer containing quaternary groups wherein said silicone polymer comprises silicone blocks with greater than about 200 siloxane units; and
b) a gel matrix comprising:
i) a cationic surfactant;
ii) a high melting point fatty compound; and
iii) an aqueous carrier.

2. The hair conditioning composition of claim 1, wherein said silicone polymer is present in an amount of from about 0.1% to about 15% by weight of the composition.

3. The hair conditioning composition of claim 1, wherein said silicone polymer is present in an amount of from about 0.25% to about 10% by weight of the composition.

4. The hair conditioning composition of claim 1, wherein said silicone polymer is present in an amount of from about 0.5% to about 5% by weight of the composition.

5. The hair conditioning composition of claim 1, wherein said silicone polymer corresponds to the following formula:
A¹-B-(A²-B)ₘ-A¹
wherein,
B is a silicone block with greater than about 200 siloxane units;
A² is a non-silicone block containing quaternary nitrogen groups;
A¹ is an end group; and
m is an integer 0 or greater; and
wherein when m is 2 or greater, each A² independently can be the same or different.

6. The hair conditioning composition of claim 1, wherein said silicone polymer corresponds to the following formula: 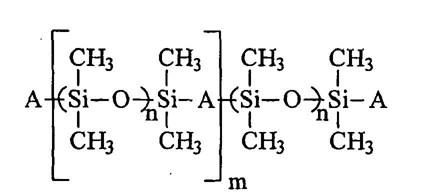 wherein,
A is a group which contains at least one quaternary nitrogen group and is linked to the silicone atoms of the silicone block by a silicon-carbon bond, wherein each A independently can be the same or different;
m is an integer 0 or greater; and
n is an integer greater than about 200.

7. The hair conditioning composition of claim 6, wherein said n is an integer of from about 300 to about 500.

8. The hair conditioning composition of claim 1, wherein said silicone polymer corresponds to the following formula: 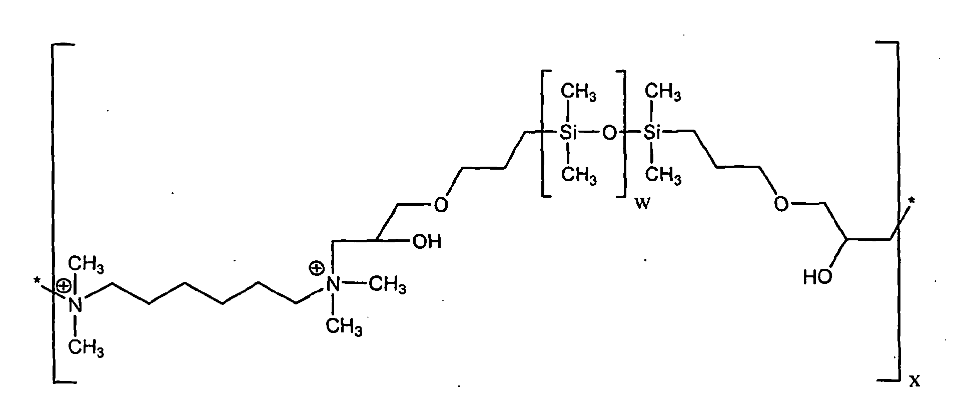 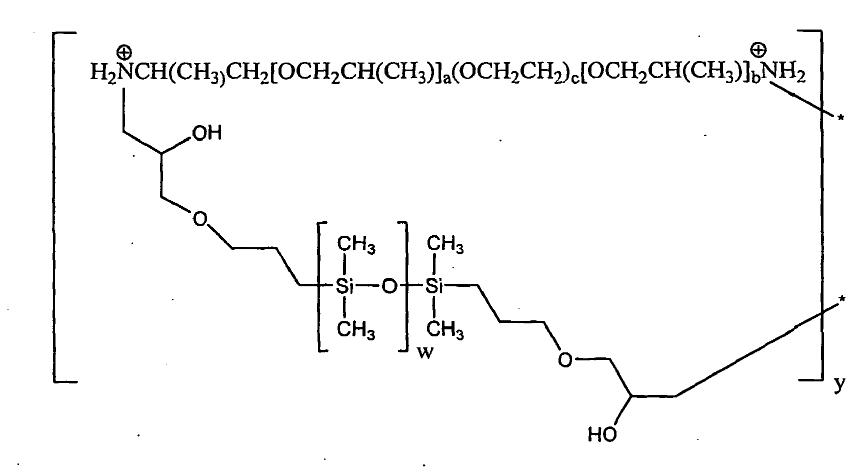 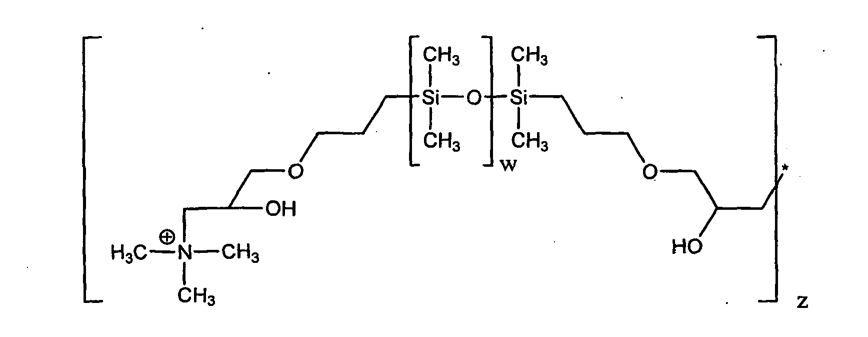 wherein,
x, y, and z represent mole fractions of the respective components;
a + b is an integer less than about 20;
c is an integer less than about 200;
w is an integer greater than about 200; and
A⁻ is an organic or inorganic anion.

9. The hair conditioning composition of claim 8, wherein said w is an integer from about 300 to about 500.

10. The hair conditioning composition of claim 1, wherein said cationic surfactant is present in an amount of from about 0.1% to about 10% by weight of the composition.

11. The hair conditioning composition of claim 1, wherein said silicone polymer is added to the formulation as a blend with a relatively lower molecular weight material, and wherein said relatively lower molecular weight material is selected from the group consisting of silicones, oxygen-containing solvents, and silicone copolyols.

12. A method of providing improved conditioning benefits to hair and/or skin, said method comprising the step of applying to said hair and/or skin the conditioning composition of claim 1.

13. A hair conditioning composition, comprising:
a) a silicone polymer containing quaternary groups, wherein said silicone polymer comprises silicone blocks with greater than about 200 siloxane units;
b) at least one additional silicone-based component selected from the group consisting of silicone emulsions, amino silicones, silicone copolyols, and silicone-based quaternary ammonium compounds that are different from said silicone polymer containing quaternary groups; and
c) a gel matrix comprising:
i) a cationic surfactant;
ii) a high melting point fatty compound; and
iii) an aqueous carrier.

14. The hair conditioning composition of claim 13, wherein said at least one additional silicone-based component comprises two components selected from said group consisting of silicone emulsions, amino silicones, silicone copolyols, and silicone-based quaternary ammonium compounds that are different from said silicone polymer containing quaternary groups.

15. The hair conditioning composition of claim 13, wherein said at least one additional silicone-based component comprises three components selected from said group consisting of silicone emulsions, amino silicones, silicone copolyols, and silicone-based quaternary ammonium compounds that are different from said silicone polymer containing quaternary groups.

16. The hair conditioning composition of claim 13, wherein said at least one additional silicone-based component comprises four components selected from said group consisting of silicone emulsions, amino silicones, silicone copolyols, and silicone-based quaternary ammonium compounds that are different from said silicone polymer containing quaternary groups.

17. The hair conditioning composition of claim 13, wherein said silicone polymer containing quaternary groups corresponds to the following formula:
A¹-B-(A²-B)ₘ-A¹
wherein,
B is a silicone block with greater than about 200 siloxane units;
A² is a non-silicone block containing quaternary nitrogen groups;
A¹ is an end group; and
m is an integer 0 or greater; and
wherein when m is 2 or greater, each A² independently can be the same or different.

18. The hair conditioning composition of claim 17, wherein said at least one additional silicone-based component comprises a silicone emulsion having an internal phase viscosity of greater than about 120,000,000 centistokes.

19. The hair conditioning composition of claim 18, wherein said at least one additional silicone-based component further comprises a silicone copolyol.

20. The hair conditioning composition of claim 19, wherein said at least one additional silicone-based component further comprises an amino silicone.

21. The hair conditioning composition of claim 20, wherein said amino silicone corresponds to the following formula:
(R₁)ₐG₃₋ₐ-Si-(-OSiG₂)ₙ-(-OSiG_{b}(R₁)_{2-b})ₘ-O-SiG₃₋ₐ(R₁)ₐ
wherein,
G is selected from the group consisting of hydrogen, phenyl, OH, C₁-C₈ alkyl, and methyl;
a is 0 or an integer having a value from 1 to 3;
b is 0, 1, or 2;
n is a number from 0 to 1,999;
m is an integer from 0 to 1,999;
and wherein
the sum of n and m is a number from 1 to 2,000;
a and m are not both 0;
R₁ is a monovalent radical of formula CqH₂qL in which q is an integer from 2 to 8; and L is selected from the group consisting of:
-N(R₂)CH₂-CH₂-N(R₂)₂;
-N(R₂)₂;
-N(R₂)⁺₃A⁻;
and
-N(R₂)CH₂-CH₂-NR₂H⁺A⁻,
in which R₂ is selected from the group consisting of hydrogen, phenyl, benzyl, a saturated hydrocarbon radical, and an alkyl radical containing from 1 to 20 carbon atoms; and A⁻ denotes a halide ion.

22. The hair conditioning composition of claim 17, wherein said at least one additional silicone-based component comprises an amino silicone.

23. The hair conditioning composition of claim 22, wherein said amino silicone corresponds to the following formula:
(R₁)ₐG₃₋ₐ-Si-(-OSiG₂)ₙ-(-OSiG_{b}(R₁)_{2-b})ₘ-O-SiG₃₋ₐ(R₁)ₐ
wherein,
G is selected from the group consisting of hydrogen, phenyl, OH, C₁-C₈ alkyl, and methyl;
a is 0 or an integer having a value from 1 to 3;
b is 0, 1, or 2;
n is a number from 0 to 1,999;
m is an integer from 0 to 1,999;
and wherein
the sum of n and m is a number from 1 to 2,000;
a and m are not both 0;
R₁ is a monovalent radical of formula C_{q}H_{2q}L in which q is an integer from 2 to 8; and L is selected from the group consisting of:
-N(R₂)CH₂-CH₂-N(R₂)₂;
-N(R₂)₂;
-N(R₂)+₃A⁻;
and
-N(R₂)CH₂-CH₂-NR₂H+A⁻,
in which R₂ is selected from the group consisting of bydrogen, phenyl, benzyl) a saturated hydrocarbon radical, and an alkyl radical containing from 1 to 20 carbon atoms; and A⁻ denotes a halide ion.

24. The hair conditioning composition of claim 22, wherein said at least one additional silicone-based component further comprises a silicone copolyol.

25. A merchandising system, comprising:
a hair conditioner in accordance with claim 1; and
at least one of packaging for said hair conditioner and marketing material pertaining to said hair conditioner comprising indicia and/or an image providing a communication to consumers related to hair breakage, bair fall out or hair shed, and/or hair strength.

26. A merchandising system, comprising:
a hair conditioner in accordance with claim 13; and
at least one of packaging for said hair conditioner and marketing material pertaining to said hair conditioner comprising indicia and/or an image providing a communication to consumers related to hair breakage, hair fall out or hair shed, and/or hair strength.

## Patentansprüche

1. Haarkonditionierungszusammensetzung, umfassend:
a) ein Silikonpolymer, das quartäre Gruppen enthält, wobei das Silikonpolymer Silikonblöcke mit mehr als ungefähr 200 Siloxaneinheiten umfasst; und
b) eine Gelmatrix, umfassend:
i) ein kationisches Tensid;
ii) eine Fettverbindung mit hohem Schmelzpunkt; und
iii) einen wässrigen Träger.

2. Haarkonditionierungszusammensetzung nach Anspruch 1, wobei das Silikonpolymer in einer Menge von ungefähr 0,1 Gew.-% bis ungefähr 15 Gew.-% der Zusammensetzung vorhanden ist.

3. Haarkonditionierungszusammensetzung nach Anspruch 1, wobei das Silikonpolymer in einer Menge von ungefähr 0,25 Gew.-% bis ungefähr 10 Gew.-% der Zusammensetzung vorhanden ist.

4. Haarkonditionierungszusammensetzung nach Anspruch 1, wobei das Silikonpolymer in einer Menge von ungefähr 0,5 Gew.-% bis ungefähr 5 Gew.-% der Zusammensetzung vorhanden ist.

5. Haarkonditionierungszusammensetzung nach Anspruch 1, wobei das Silikonpolymer der folgenden Formel entspricht:
A¹-B-(A²-B)ₘ-A¹
wobei
B ein Silikonblock mit mehr als ungefähr 200 Siloxaneinheiten ist;
A² ein Nicht-Silikonblock ist, der quartäre Stickstoffgruppen enthält;
A¹ eine Endgruppe ist; und
m eine ganze Zahl von 0 oder mehr ist; und
wobei, wenn m gleich 2 oder mehr ist, jedes A² unabhängig gleich oder verschieden sein kann.

6. Haarkonditionierungszusammensetzung nach Anspruch 1, wobei das Silikonpolymer der folgenden Formel entspricht: 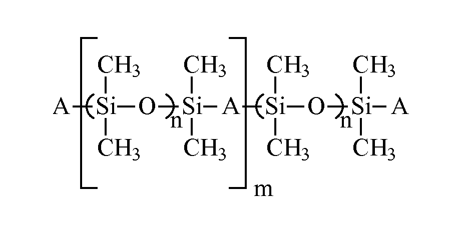 wobei
A eine Gruppe ist, die mindestens eine quartäre Stickstoffgruppe enthält und mit den Siliciumatomen des Silikonblocks über eine Silicium-Kohlenstoff-Bindung verbunden ist, wobei jedes A unabhängig gleich oder unterschiedlich sein kann;
m eine ganze Zahl von 0 oder mehr ist; und
n eine ganze Zahl von mehr als ungefähr 200 ist.

7. Haarkonditionierungszusammensetzung nach Anspruch 6, wobei das n eine ganze Zahl von ungefähr 300 bis ungefähr 500 ist.

8. Haarkonditionierungszusammensetzung nach Anspruch 1, wobei das Silikonpolymer der folgenden Formel entspricht: 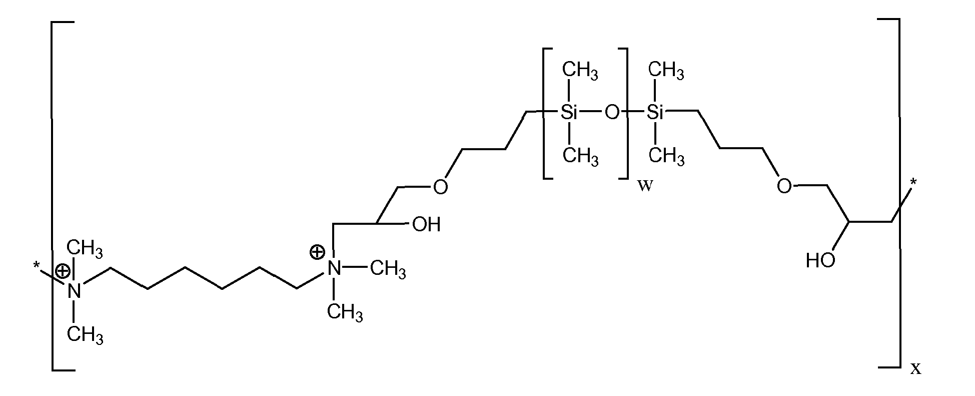 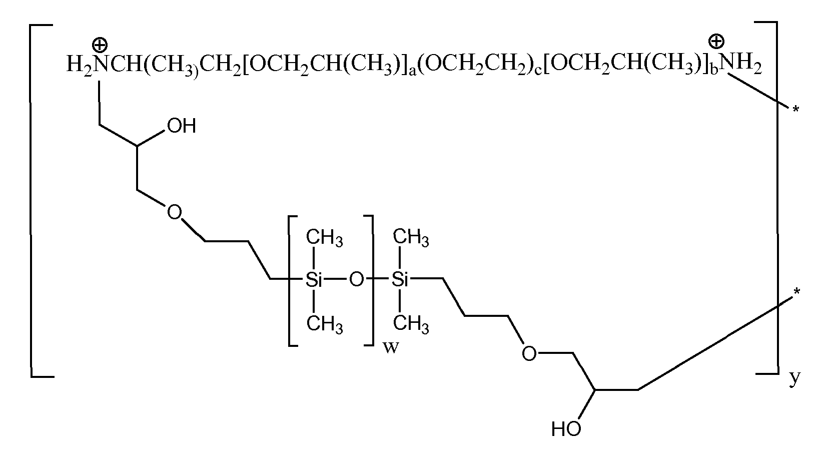 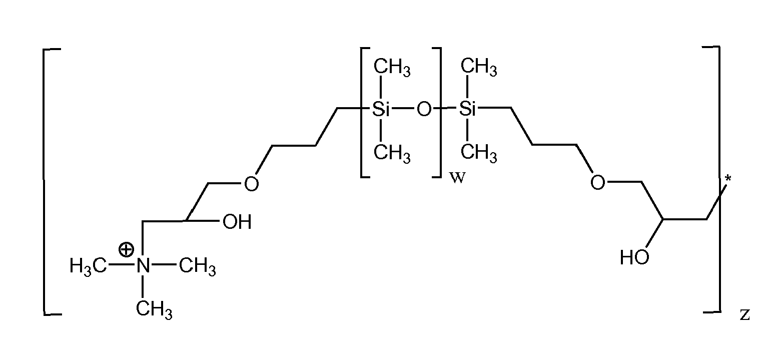 wobei x, y und z für Molfraktionen der jeweiligen Bestandteile stehen;
a + b eine ganze Zahl von weniger als ungefähr 20 ist;
c eine ganze Zahl von weniger als ungefähr 200 ist;
w eine ganze Zahl von mehr als ungefähr 200 ist; und
A- ein organisches oder anorganisches Anion ist.

9. Haarkonditionierungszusammensetzung nach Anspruch 8, wobei das w eine ganze Zahl von ungefähr 300 bis ungefähr 500 ist.

10. Haarkonditionierungszusammensetzung nach Anspruch 1, wobei das kationische Tensid in einer Menge von ungefähr 0,1 Gew.-% bis ungefähr 10 Gew.-% der Zusammensetzung vorhanden ist.

11. Haarkonditionierungszusammensetzung nach Anspruch 1, wobei das Silikonpolymer als eine Mischung mit einem relativ niedermolekularen Material zu der Formulierung gegeben wird, und wobei das relativ niedermolekulare Material ausgewählt ist aus der Gruppe bestehend aus Silikonen, sauerstoffhaltigen Lösungsmitteln und Silikoncopolyolen.

12. Verfahren zum Bereitstellen verbesserter Konditionierungsvorteile für Haar und/oder Haut, wobei das Verfahren den Schritt des Auftragens der Konditionierungszusammensetzung nach Anspruch 1 auf das Haar und/oder die Haut umfasst.

13. Haarkonditionierungszusammensetzung, umfassend:
a) ein Silikonpolymer, das quartäre Gruppen enthält, wobei das Silikonpolymer Silikonblöcke mit mehr als ungefähr 200 Siloxaneinheiten umfasst;
b) mindestens einen zusätzlichen silikonbasierten Bestandteil, ausgewählt aus der Gruppe bestehend aus Silikonemulsionen, Aminosilikonen, Silikoncopolyolen und silikonbasierten quartären Ammoniumverbindungen, die sich von dem Silikonpolymer, das quartäre Gruppen enthält, unterscheiden; und
c) eine Gelmatrix, umfassend:
i) ein kationisches Tensid;
ii) eine Fettverbindung mit hohem Schmelzpunkt; und
iii) einen wässrigen Träger.

14. Haarkonditionierungszusammensetzung nach Anspruch 13, wobei der mindestens eine zusätzliche silikonbasierte Bestandteil zwei Bestandteile umfasst, die ausgewählt sind aus der Gruppe bestehend aus Silikonemulsionen, Aminosilikonen, Silikoncopolyolen und silikonbasierten quartären Ammoniumverbindungen, die sich von dem Silikonpolymer, das quartäre Gruppen enthält, unterscheiden.

15. Haarkonditionierungszusammensetzung nach Anspruch 13, wobei der mindestens eine zusätzliche silikonbasierte Bestandteil drei Bestandteile umfasst, die ausgewählt sind aus der Gruppe bestehend aus Silikonemulsionen, Aminosilikonen, Silikoncopolyolen und silikonbasierten quartären Ammoniumverbindungen, die sich von dem Silikonpolymer, das quartäre Gruppen enthält, unterscheiden.

16. Haarkonditionierungszusammensetzung nach Anspruch 13, wobei der mindestens eine zusätzliche silikonbasierte Bestandteil vier Bestandteile umfasst, die ausgewählt sind aus der Gruppe bestehend aus Silikonemulsionen, Aminosilikonen, Silikoncopolyolen und silikonbasierten quartären Ammoniumverbindungen, die sich von dem Silikonpolymer, das quartäre Gruppen enthält, unterscheiden.

17. Haarkonditionierungszusammensetzung nach Anspruch 13, wobei das Silikonpolymer, das quartäre Gruppen enthält, der folgenden Formel entspricht:
A¹-B-(A²-B)ₘ^{-A1}
wobei
B ein Silikonblock mit mehr als ungefähr 200 Siloxaneinheiten ist;
A² ein Nicht-Silikonblock ist, der quartäre Stickstoffgruppen enthält,
A¹ eine Endgruppe ist; und
m eine ganze Zahl von 0 oder mehr ist; und
wobei, wenn m gleich 2 oder größer ist, jedes A² unabhängig gleich oder verschieden sein kann.

18. Haarkonditionierungszusammensetzung nach Anspruch 17, wobei der mindestens eine zusätzliche silikonbasierte Bestandteil eine Silikonemulsion umfasst, die eine interne Phasenviskosität von mehr als ungefähr 120 m²/s (120.000.000 Centistoke) aufweist.

19. Haarkonditionierungszusammensetzung nach Anspruch 18, wobei der mindestens eine zusätzliche silikonbasierte Bestandteil ferner ein Silikoncopolyol umfasst.

20. Haarkonditionierungszusammensetzung nach Anspruch 19, wobei der mindestens eine zusätzliche silikonbasierte Bestandteil ferner ein Aminosilikon umfasst.

21. Haarkonditionierungszusammensetzung nach Anspruch 20, wobei das Aminosilikon der folgenden Formel entspricht:
(R₁)aG₃₋ₐ-Si-(-OSiG₂)ₙ-(-OSiG_{b}(R₁)_{2-b})ₘ-O-SiG₃₋ₐ(R₁)ₐ
wobei
G ausgewählt ist aus der Gruppe bestehend aus Wasserstoff, Phenyl, OH, C₁-C₈-Alkyl und Methyl;
a 0 oder eine ganze Zahl mit einem Wert von 1 bis 3 ist;
b 0, 1 oder 2 ist;
n eine Zahl von 0 bis 1.999 ist;
m eine ganze Zahl von 0 bis 1.999 ist;
und wobei
die Summe von n und m eine Zahl von 1 bis 2.000 ist;
a und m nicht beide 0 sind;
R₁ ein einwertiges Radikal der Formel C_{q}H_{2q}L ist, wobei q eine ganze Zahl von 2 bis 8 ist; und L ausgewählt ist aus der Gruppe, bestehend aus:
-N(R₂)CH₂-CH₂-N(R₂)₂;
-N(R₂)₂;
-N(R₂)⁺₃A⁻;
und
-N(R₂)CH₂-CH₂-NR₂H⁺A⁻,
wobei R₂ ausgewählt ist aus der Gruppe bestehend aus Wasserstoff, Phenyl, Benzyl, einem gesättigten Kohlenwasserstoffradikal und einem Alkylradikal, das von 1 bis 20 Kohlenstoffatome enthält; und A⁻ für ein Halogenidion steht.

22. Haarkonditionierungszusammensetzung nach Anspruch 17, wobei der mindestens eine zusätzliche silikonbasierte Bestandteil ein Aminosilikon umfasst.

23. Haarkonditionierungszusammensetzung nach Anspruch 22, wobei das Aminosilikon der folgenden Formel entspricht:
(R₁)aG₃₋ₐ-Si-(-OSiG₂)ₙ-(-OSiG_{b}(R₁)_{2-b})ₘ-O-SiG₃₋ₐ(R₁)ₐ
wobei
G ausgewählt ist aus der Gruppe bestehend aus Wasserstoff, Phenyl, OH, C₁-C₈-Alkyl und Methyl;
a 0 oder eine ganze Zahl mit einem Wert von 1 bis 3 ist;
b 0, 1 oder 2 ist;
n eine Zahl von 0 bis 1.999 ist;
m eine ganze Zahl von 0 bis 1.999 ist;
und wobei
die Summe von n und m eine Zahl von 1 bis 2.000 ist;
a und m nicht beide 0 sind;
R₁ ein einwertiges Radikal der Formel C_{q}H_{2q}L ist, wobei q eine ganze Zahl von 2 bis 8 ist; und L ausgewählt ist aus der Gruppe bestehend aus:
-N(R₂)CH₂-CH₂-N(R₂)₂;
-N(R₂)₂;
-N(R₂)⁺₃A⁻;
und
-N(R₂)CH₂-CH₂-NR₂H⁺A⁻,
wobei R₂ ausgewählt ist aus der Gruppe bestehend aus Wasserstoff, Phenyl, Benzyl, einem gesättigten Kohlenwasserstoffradikal und einem Alkylradikal, das von 1 bis 20 Kohlenstoffatome enthält; und A⁻ für ein Halogenidion steht.

24. Haarkonditionierungszusammensetzung nach Anspruch 22, wobei der mindestens eine zusätzliche silikonbasierte Bestandteil ferner ein Silikoncopolyol umfasst.

25. Vermarktungssystem, umfassend:
ein Haarkonditionierungsrmittel nach Anspruch 1; und
mindestens entweder eine Verpackung für das Haarkonditionierungsmittel und/oder Marketingmaterial bezüglich des Haarkonditionierungsmittels, umfassend ein Zeichen und/oder ein Bild, das Verbrauchern eine Mitteilung in Bezug auf Haarbruch, Haarausfall oder Haarverlust und/oder Haarkräftigkeit bereitstellt.

26. Vermarktungssystem, umfassend:
ein Haarkonditionierungsmittel nach Anspruch 13; und
mindestens entweder eine Verpackung für das Haarkonditionierungsmittel und/oder Marketingmaterial bezüglich des Haarkonditionierungsmittels, umfassend ein Zeichen und/oder ein Bild, das Verbrauchern eine Mitteilung in Bezug auf Haarbruch, Haarausfall oder Haarverlust und/oder Haarkräftigkeit bereitstellt.

## Revendications

1. Composition de conditionnement des cheveux comprenant:
a) un polymère de silicone contenant des groupes quaternaires dans laquelle ledit polymère de silicone comprend des blocs de silicone avec plus d'environ 200 motifs siloxane ; et
b) une matrice de gel comprenant :
i) un agent tensioactif cationique ;
ii) un composé gras à point de fusion élevé ; et
iii) un véhicule aqueux.

2. Composition de conditionnement des cheveux selon la revendication 1, dans laquelle ledit polymère de silicone est présent en une quantité d'environ 0,1 % à environ 15 % en poids de la composition.

3. Composition de conditionnement des cheveux selon la revendication 1, dans laquelle ledit polymère de silicone est présent en une quantité d'environ 0,25 % à environ 10 % en poids de la composition.

4. Composition de conditionnement des cheveux selon la revendication 1, dans laquelle ledit polymère de silicone est présent en une quantité d'environ 0,5 % à environ 5 % en poids de la composition.

5. Composition de conditionnement des cheveux selon la revendication 1, dans laquelle ledit polymère de silicone correspond à la formule suivante :
A¹-B-(A²-B)ₘ-A¹
dans laquelle,
B est un bloc de silicone avec plus d'environ 200 motifs siloxane ;
A² est un bloc non-silicone contenant des groupes azotés quaternaires ;
A¹ est un groupe terminal ; et
m est un nombre entier 0 ou plus grand ; et
dans laquelle lorsque m est 2 ou plus, chaque A² peut être indépendamment identique ou différent.

6. Composition de conditionnement des cheveux selon la revendication 1, dans laquelle ledit polymère de silicone correspond à la formule suivante : 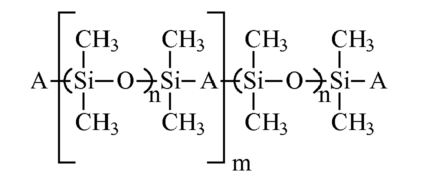 dans laquelle,
A est un groupe qui contient au moins un groupe azote quaternaire et est lié aux atomes de silicium du bloc de silicone par une liaison silicium-carbone, dans laquelle chaque A peut indépendamment être identique ou différent ;
m est un nombre entier 0 ou plus grand ; et
n est un nombre entier supérieur à environ 200.

7. Composition de conditionnement des cheveux selon la revendication 6, dans laquelle ledit n est un nombre entier allant d'environ 300 à environ 500.

8. Composition de conditionnement des cheveux selon la revendication 1, dans laquelle ledit polymère de silicone correspond à la formule suivante : 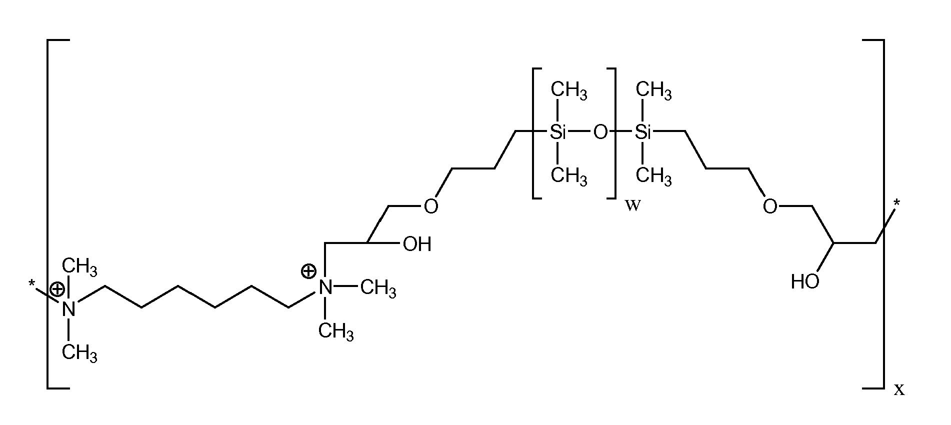 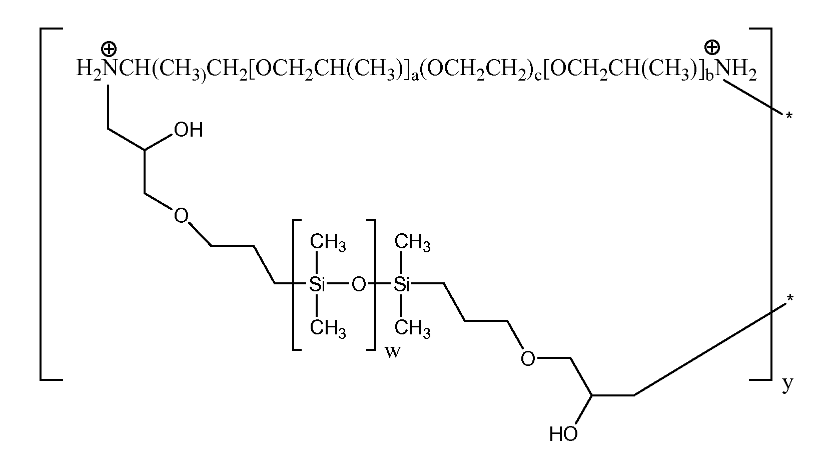 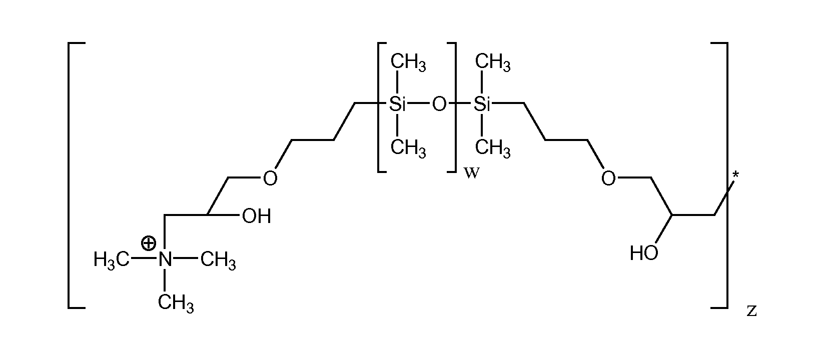 dans laquelle,
x, y, et z représentent les fractions molaires des composants respectifs ; a + b est un nombre entier inférieur à environ 20 ;
c est un nombre entier inférieur à environ 200 ;
w est un nombre entier supérieur à environ 200 ; et
A- est un anion organique ou inorganique.

9. Composition de conditionnement des cheveux selon la revendication 8, dans laquelle ledit w est un nombre entier allant d'environ 300 à environ 500.

10. Composition de conditionnement des cheveux selon la revendication 1, dans laquelle ledit agent tensioactif cationique est présent en une quantité d'environ 0,1 % à environ 10 % en poids de la composition.

11. Composition de conditionnement des cheveux selon la revendication 1, dans laquelle ledit polymère de silicone est ajouté à la formulation en tant que mélange avec un matériau à masse moléculaire relativement plus basse, et dans laquelle ledit matériau à masse moléculaire relativement plus basse est choisi dans le groupe constitué de silicones, solvants contenant de l'oxygène, et copolyols de silicone.

12. Procédé de fourniture d'effets bénéfiques de conditionnement amélioré aux cheveux et/ou à la peau, ledit procédé comprenant l'étape consistant à appliquer sur lesdits cheveux et/ou ladite peau la composition de conditionnement selon la revendication 1.

13. Composition de conditionnement des cheveux comprenant:
a) un polymère de silicone contenant des groupes quaternaires, dans laquelle ledit polymère de silicone comprend des blocs de silicone avec plus d'environ 200 motifs siloxane ;
b) au moins un composant à base de silicone supplémentaire choisi dans le groupe constitué d'émulsions de silicone, aminosilicones, copolyols de silicone, et composés d'ammonium quaternaire à base de silicone qui sont différents dudit polymère de silicone contenant des groupes quaternaires ; et
c) une matrice de gel comprenant :
i) un agent tensioactif cationique ;
ii) un composé gras à point de fusion élevé ; et
iii) un véhicule aqueux.

14. Composition de conditionnement des cheveux selon la revendication 13, dans laquelle ledit au moins un composant à base de silicone supplémentaire comprend deux composants choisis parmi ledit groupe constitué d'émulsions de silicone, aminosilicones, copolyols de silicone, et composés d'ammonium quaternaire à base de silicone qui sont différents dudit polymère de silicone contenant des groupes quaternaires.

15. Composition de conditionnement des cheveux selon la revendication 13, dans laquelle ledit au moins un composant à base de silicone supplémentaire comprend trois composants choisis parmi ledit groupe constitué d'émulsions de silicone, aminosilicones, copolyols de silicone, et composés d'ammonium quaternaire à base de silicone qui sont différents dudit polymère de silicone contenant des groupes quaternaires.

16. Composition de conditionnement des cheveux selon la revendication 13, dans laquelle ledit au moins un composant à base de silicone supplémentaire comprend quatre composants choisis parmi ledit groupe constitué d'émulsions de silicone, aminosilicones, copolyols de silicone, et composés d'ammonium quaternaire à base de silicone qui sont différents dudit polymère de silicone contenant des groupes quaternaires.

17. Composition de conditionnement des cheveux selon la revendication 13, dans laquelle ledit polymère de silicone contenant des groupes quaternaires correspond à la formule suivante :
A¹-B-(A²-B)ₘ-A¹
dans laquelle,
B est un bloc de silicone avec plus d'environ 200 motifs siloxane ;
A² est un bloc non-silicone contenant des groupes azotés quaternaires ;
A¹ est un groupe terminal ; et
m est un nombre entier 0 ou plus grand ; et
dans laquelle lorsque m est 2 ou plus, chaque A² peut indépendamment être identique ou différent.

18. Composition de conditionnement des cheveux selon la revendication 17, dans laquelle ledit au moins un composant à base de silicone supplémentaire comprend une émulsion de silicone ayant une viscosité de phase interne supérieure à environ 120 m²/s (120 000 000 centistokes).

19. Composition de conditionnement des cheveux selon la revendication 18, dans laquelle ledit au moins un composant à base de silicone supplémentaire comprend en outre un copolyol de silicone.

20. Composition de conditionnement des cheveux selon la revendication 19, dans laquelle ledit au moins un composant à base de silicone supplémentaire comprend en outre une aminosilicone.

21. Composition de conditionnement des cheveux selon la revendication 20, dans laquelle ladite aminosilicone correspond à la formule suivante :
(R₁)aG₃₋ₐ-Si-(-OSiG₂)ₙ-(-OSiG_{b}(R₁)_{2-b})ₘ-O-SiG₃₋ₐ(R₁)ₐ
dans laquelle,
G est choisi dans le groupe constitué d'hydrogène, phényle, OH, alkyle en C₁ à C₈, et méthyle ;
a est 0 ou un nombre entier ayant une valeur de 1 à 3 ;
b est 0, 1, ou 2 ;
n est un nombre de 0 à 1999 ;
m est un nombre entier allant de 0 à 1999 ;
et dans laquelle
la somme de n et m est un nombre de 1 à 2000 ;
a et m ne sont pas l'un et l'autre 0 ;
R₁ est un radical monovalent de formule C_{q}H_{2q}L dans lequel q est un nombre entier allant de 2 à 8 ; et L est choisi dans le groupe constitué de :
-N(R₂)CH₂-CH₂-N(R₂)₂;
-N(R₂)₂;
-N(R₂)⁺₃A⁻ ;
et
-N(R₂)CH₂-CH₂-NR₂H⁺A⁻,
dans lesquels R₂ est choisi dans le groupe constitué d'hydrogène, phényle, benzyle, un radical hydrocarbure saturé, et un radical alkyle contenant de 1 à 20 atomes de carbone ; et A⁻ désigne un ion halogénure.

22. Composition de conditionnement des cheveux selon la revendication 17, dans laquelle ledit au moins un composant à base de silicone supplémentaire comprend une aminosilicone.

23. Composition de conditionnement des cheveux selon la revendication 22, dans laquelle ladite aminosilicone correspond à la formule suivante :
(R₁)aG₃₋ₐ-Si-(-OSiG₂)ₙ-(-OSiG_{b}(R₁)_{2-b})ₘ -O-SiG₃₋ₐ(R₁)ₐ
dans laquelle,
G est choisi dans le groupe constitué d'hydrogène, phényle, OH, alkyle en C₁ à C₈, et méthyle ;
a est 0 ou un nombre entier ayant une valeur de 1 à 3 ;
b est 0, 1, ou 2 ;
n est un nombre de 0 à 1999 ;
m est un nombre entier allant de 0 à 1999 ;
et dans laquelle
la somme de n et m est un nombre de 1 à 2000 ;
a et m ne sont pas l'un et l'autre 0 ;
R1 est un radical monovalent de formule C_{q}H_{2q}L dans laquelle q est un nombre entier allant de 2 à 8 ; et L est choisi dans le groupe constitué de :
-N(R₂)CH₂-CH₂-N(R₂)₂ ;
-N(R₂)₂ ;
-N(R₂)⁺₃A⁻ ;
et
-N(R₂)CH₂-CH₂-NR₂H⁺A⁻,
dans lesquels R₂ est choisi dans le groupe constitué d'hydrogène, phényle, benzyle, un radical hydrocarbure saturé, et un radical alkyle contenant de 1 à 20 atomes de carbone ; et A⁻ désigne un ion halogénure.

24. Composition de conditionnement des cheveux selon la revendication 22, dans laquelle ledit au moins un composant à base de silicone supplémentaire comprend en outre un copolyol de silicone.

25. Système de marchandisage, comprenant :
un conditionneur pour les cheveux selon la revendication 1 ; et
au moins un élément parmi un conditionnement pour ledit conditionneur pour les cheveux et un matériau de marketing en rapport avec ledit conditionneur pour les cheveux comprenant des estampilles commerciales et/ou une image fournissant une communication à des consommateurs se rapportant à une cassure des cheveux, une chute des cheveux ou une perte de cheveux, et/ou la solidité des cheveux.

26. Système de marchandisage, comprenant :
un conditionneur pour les cheveux selon la revendication 13 ; et
au moins un élément parmi un conditionnement pour ledit conditionneur pour les cheveux et un matériau de marketing en rapport avec ledit conditionneur pour les cheveux comprenant des estampilles commerciales et/ou une image fournissant une communication à des consommateurs se rapportant à une cassure des cheveux, une chute des cheveux ou une perte de cheveux, et/ou la solidité des cheveux.
